(19) Europäisches Patentamt
European Patent Office
Office européen des brevets

(11) Veröffentlichungsnummer : **0 008 768**
**B1**

(12) # EUROPÄISCHE PATENTSCHRIFT

(45) Veröffentlichungstag der Patentschrift : **15.04.81**

(51) Int. Cl.³ : **C 07 C127/22**, C 07 D333/38, C 07 D213/82, A 01 N 47/34

(21) Anmeldenummer : **79103185.9**

(22) Anmeldetag : **29.08.79**

(54) **Aroylharnstoffe, Verfahren zu ihrer Herstellung sowie ihre Verwendung in insektiziden und akariziden Mitteln.**

(30) Priorität : **11.09.78 DE 2839462**

(43) Veröffentlichungstag der Anmeldung : **19.03.80 (Patentblatt 80/06)**

(45) Bekanntmachung des Hinweises auf die Patenterteilung : **15.04.81 Patentblatt 81/15**

(84) Benannte Vertragsstaaten : **AT BE CH DE FR GB IT LU NL SE**

(56) Entgegenhaltungen : **DE - A - 2 552 933**

(73) Patentinhaber : **BASF Aktiengesellschaft
Carl-Bosch-Strasse 38
D-6700 Ludwigshafen (DE)**

(72) Erfinder : **Baumann, Annegrit, Dr.
Im Sennteich 26
D-6800 Mannheim (DE)**
Erfinder : **Kiehs, Karl, Dr.
Sudetenstrasse 22
D-6840 Lampertheim (DE)**
Erfinder : **Adolphi, Heinrich, Dr., Dipl.-Biologe
Kalmitweg 11
D-6703 Limburgerhof (DE)**
Erfinder : **Koenig, Karl-Heinz, Dr.
Pierstrasse 8 a
D-6710 Frankenthal (DE)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Imprimerie Jouve, 18, rue St-Denis, 75001 Paris, France

EP 0 008 768 B1

# Aroylharnstoffe, Verfahren zu ihrer Herstellung sowie ihre Verwendung in insektiziden und akariziden Mitteln

Die vorliegende Erfindung betrifft Aroylharnstoffe, ein Verfahren zu ihrer Herstellung, insektizide und akarizide Mittel, die diese Harnstoffe als Wirkstoffe enthalten, sowie ein Verfahren zur Bekämpfung von Insekten und Milben mit diesen Wirkstoffen.

Insektizid wirksame N-Benzoyl-N'-phenyl-harnstoffe sind aus der DE-A 2 123 236, der DE-A 2 531 279 und der DE-A 2 601 780 bekannt. Diese Wirkstoffe tragen am Phenylring im wesentlichen Substituenten aus den Gruppen Halogen, Alkyl, Halogenalkyl bzw. Halogenalkoxy und Halogenalkylthio. Sie eignen sich zur Bekämpfung von Insekten, wie Raupen und Käfer.

Weiterhin ist aus J. Agric. Food Chem. *26,* 164-166 (1978) bekannt, daß N-(2,6-Difluorbenzoyl)-N'-pyridinyl-harnstoffe entwicklungshemmende Wirkung bei Musca domestica und Spodoptera frugiperda zeigen.

Schließlich sind aus der DE-A 2 552 933 Hydroxypolyhalogenisopropyl-substituierte Ureidoverbindungen bekannt, welche als Arzneimittel Anwendung finden können.

Es wurde gefunden, daß Aroylharnstoffe der Formel I

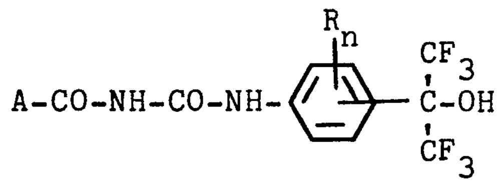 I,

in der

A einen durch Halogen, unverzeigtes oder verzweigtes Alkyl mit 1 bis 4 Kohlenstoffatomen oder unverzweigtes oder verzweigtes Alkoxy mit 1 bis 4 Kohlenstoffatomen einfach oder mehrfach substituierten Phenyl-, Thienyl- oder Pyridinyl-3-rest,

R unverzweigtes oder verzweigtes Alkyl mit 1 bis 4 Kohlenstoffatomen oder unverzweigtes oder verzweigtes Alkoxy mit 1 bis 4 Kohlenstoffatomen und n 0, 1 oder 2 bedeuten, starke insektizide Eigenschaften besitzen. Überraschenderweise sind sie außerdem akarizid wirksam und besitzen damit eine Eigenschaft, die bisher in der Wirkstoffklasse der Benzoylharnstoffe nicht beobachtet wurde.

In der Formel I steht A für einen durch Halogen, unverzweigtes oder verzweigtes Alkyl mit 1 bis 4 Kohlenstoffatomen oder unverzweigtes oder verzweigtes Alkoxy mit 1 bis 4 Kohlenstoffatomen einfach oder mehrfach substituierten Phenyl-, Pyridinyl-3- oder Thienylrest, wie den Thienyl-3-rest. Als Substituenten kommen beispielsweise Fluor, Chlor, Brom, Methyl, Ethyl, n-Propyl, i-Propyl, i-Butyl, sec.-Butyl, Methoxy, Ethoxy oder Propoxy- reste in Betracht. Bevorzugte Substituenten sind Fluor, Chlor, Methyl oder Methoxy. Die aromatischen Ringe tragen vorzugsweise bis zu zwei Substituenten.

R in der Formel I bedeutet unverzweigtes oder verzweigtes Alkyl mit 1 bis 4 Kohlenstoffatomen, beispielsweise Methyl, Ethyl, i-Propyl, i-Butyl, sec.-Butyl, insbesondere Methyl, oder unverzweigtes oder verzweigtes Alkoxy mit 1 bis 4 Kohlenstoffatomen, beispielsweise Methoxy, Ethoxy, n-Propoxy, i-Propoxy oder einen Butoxyrest, vorzugsweise Methoxy oder Ethoxy.

Bevorzugte Aroylharnstoffe der Formel I sind solche, bei denen A für einen durch Halogen einfach oder zweifach substituierten Phenyl-, Thienyl- oder Pyridinyl-3-rest, und n für O steht.

Außer den in den Beispielen genannten Verbindungen sollen folgende weitere Verbindungen der Formel I genannt werden :

| A | Rn |
|---|---|
| 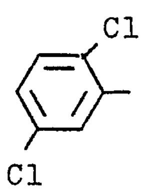 | 2,6-Dimethyl |
| 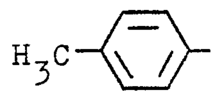 | 2,6-Dimethyl |

| A | Rn |
|---|---|
| 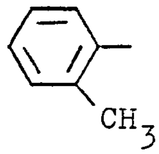 | 2,6-Dimethyl |
| 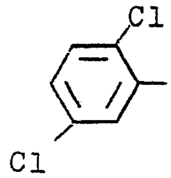 | 2,5-Dimethyl |
| 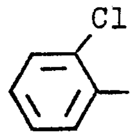 | 2,5-Dimethyl |
| 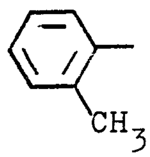 | 2-Methyl |
| 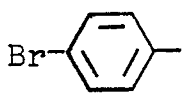 | 2-Methyl |
| 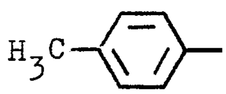 | 2-Methyl |
| 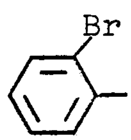 | 2-Isopropyl |
| 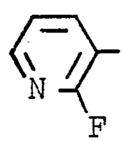 | - |
| 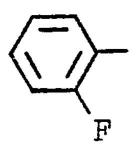 | 2-Methyl |
| 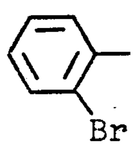 | 2-Methoxy |
| 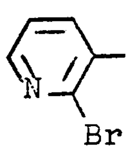 | - |
| 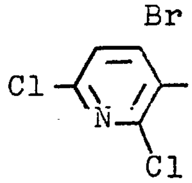 | - |

| A | $R_n$ |
| --- | --- |
| 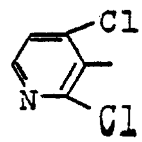 | - |
| 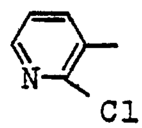 | 2-Methoxy |
| 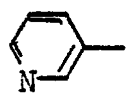 | - |
| 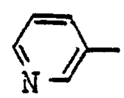 | 2-Methyl |
| 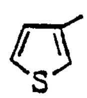 | - |
| 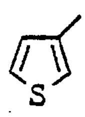 | 2-Methyl |
| 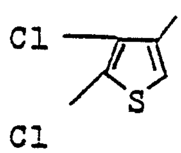 | - |
| 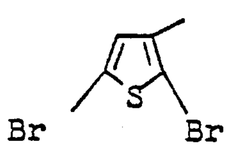 | - |
| 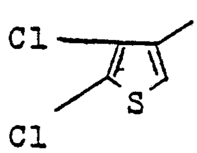 | 2-Methyl |

| A | $R_n$ |
| --- | --- |
| 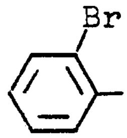 | 4-Methyl |
| 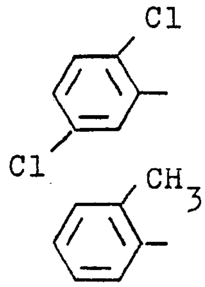 | 4-Methyl |
| | 4-Methyl |
| 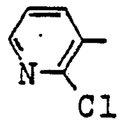 | 4-Ethyl |
| 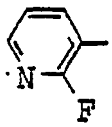 | 4-Methyl |
| 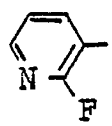 | 4-Ethyl |
| 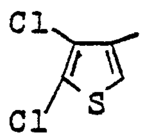 | 4-Methyl |
| 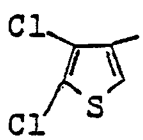 | 4-Ethyl |
| 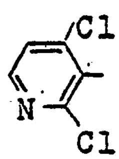 | 4-Methyl |
| 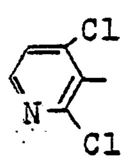 | 4-Ethyl |

Weiterhin wurde gefunden, daß die neuen Aroylharnstoffe der Formel I durch Umsetzung von Isocyanaten der Formel II

A—CO—NCO (II)

in der A die obengenannten Bedeutungen hat, mit substituierten Anilinen der Formel III

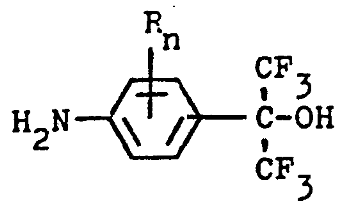

(III)

in der R und n die obengenannten Bedeutungen haben, in Gegenwart eines inerten organischen Lösungsmittels bei einer Temperatur im Bereich zwischen 0 und 80 °C erhalten werden.

Dabei kommen als inerte organische Solventien aliphatische und aromatische, gegebenenfalls chlorierte oder nitrierte Kohlenwasserstoffe, wie Benzol, Toluol, Xylole, Chlorbenzole, Benzin, Tetrachlorkohlenstoff, 1,2-Dichlorethan, Methylenchlorid, Chloroform, Nitromethan, acyclische und cyclische Ether, wie Diethylether, Dibutylether, Tetrahydrofuran, Dioxan, acyclische und cyclische Ketone, wie Aceton, Methylethylketon, Methylisopropylketon, Cyclohexanon, außerdem Nitrile, wie Acetonitril, Benzonitril, in Betracht. Auch Gemische dieser Lösungsmittel können verwendet werden.

Die Reaktionstemperatur kann innerhalb eines größeren Bereiches variiert werden. Sie liegt in der Regel zwischen 0 und 80 °C. Die Umsetzung verläuft aufgrund der exothermen Reaktion bei einer Temperatur zwischen 20 und 60 °C, wenn nicht zusätzlich gekühlt wird.

Man läßt die Umsetzung im allgemeinen bei Normaldruck ablaufen.

Zur Durchführung der Verfahren setzt man die Reaktionskomponenten vorzugsweise in äquimolaren Verhältnissen ein. Ein Überschuß der einen oder anderen Komponente bringt keine wesentlichen Vorteile. Im allgemeinen wird das substituierte Anilin der Formel III zusammen mit dem Lösungs-oder Verdünnungsmittel vorgelegt. Die Umsetzung verläuft praktisch quantitativ. Nach mehrstündiger Reaktionsdauer, in der Regel nach 2 Stunden, wird das Produkt abgesaugt und unter vermindertem Druck getrocknet.

Die erfindungsgemäßen Verbindungen fallen als Festprodukte an, die in der Regel analysenrein sind, andernfalls aber durch Umkristallisieren gereinigt werden können. Zu ihrer Charakterisierung dienen Elementaranalyse und Schmelzpunkt.

Die Isocyanate der Formel II können durch Umsetzung der entsprechenden primären Amide mit Oxalylchlorid nach folgender Reaktionsgleichung hergestellt werden :

$$A\text{-}CO\text{-}NH_2 + Cl\text{-}CO\text{-}CO\text{-}Cl \longrightarrow A\text{-}CO\text{-}NCO + CO + 2\ HCl$$

Dabei hat A die obengenannten Bedeutungen (J. Org. Chem. *28,* 1805-1811 (1963)).

Die substituierten Aniline der Formel III sind bekannt oder nach dem in J. Org. Chem. *30,* 1001-1003 (1965)) beschriebenen Verfahren durch Umsetzung von Hexafluoraceton mit einem Anilin der Formel

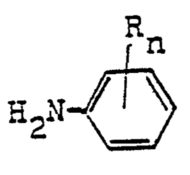

,

in der R und n die oben genannten Bedeutungen haben, herstellbar.

Die Aroylharnstoffe der Formel I können auch durch Umsetzung von Amiden der Formel $A\text{-}CO\text{-}NH_2$, in der A die oben genannten Bedeutungen hat, mit Isocyanaten der Formel

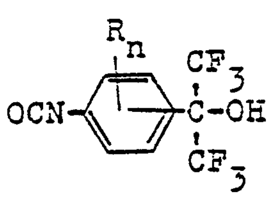

,

in der R und n die oben genannten Bedeutungen haben, erhalten werden. Die Umsetzung kann in Pyridin in Gegenwart einer katalytischen Menge Natrium bei einer Temperatur im Bereich zwischen 40 und 120 °C oder in Tetrahydrofuran mit Natriumhydrid als Base bei einer Temperatur im Bereich zwischen —20 °C und der Rückflußtemperatur des Lösungsmittels durchgeführt werden.

Die folgenden Beispiele erläutern die Herstellung der erfindungsgemäßen Aroylharnstoffe der Formel I.

### Beispiel 1

0,05 Mol 4-(1-Hydroxy-1-trifluormethyl-2,2,2-trifluor-ethyl)-anilin werden in 150 ml absolutem Toluol vorgelegt. Dazu werden 0,055 Mol 2,6-Difluorbenzoyl-isocyanat getropft. Die Temperatur steigt auf 27 °C an. Es wird noch drei Stunden auf 50 °C erhitzt, dann wird warm abgesaugt und bei 50 °C unter vermindertem Druck getrocknet. Man erhält 19,5 g N-(2,6-Difluorbenzoyl)-N'-[4-(1-Hydroxy-1-trifluormethyl-2,2,2-trifluor-ethyl)-phenyl]-harnstoff ; Ausbeute 95 % d. Th. ; Fp : 205-207 °C.

| | C | H | N | F |
|---|---|---|---|---|
| ber. : | 46.17 | 2.28 | 6.33 | 34.37 |
| gef. : | 46.1 | 2,4 | 6.1 | 34.4 |

### Beispiel 2

0,035 Mol 4-(1-Hydroxy-1-trifluormethyl-2,2,2-trifluorethyl)-anilin werden in 100 ml absolutem Toluol vorgelegt. Dazu wurden 0,04 Mol 4-Chlor-thenoyl-3-isocyanat in 30 ml Toluol getropft, wobei die Temperatur auf 35 °C ansteigt. Nach 3 Stunden Rühren bei 50 °C wird der Niederschlag warm abgesaugt, mit Ether gewaschen und unter vermindertem Druck bei 50 °C getrocknet. Man erhält 11,8 g analysenreinen N-(4-Chlor-thenoyl-3)-N'-[4-(1-hydroxy-1-trifluormethyl-2,2,2-trifluor-ethyl)-phenyl]-harnstoff ; Ausbeute : 76 % d. Th. ; Fp. : 176-179 °C.

| | C | H | N | S | Cl | F |
|---|---|---|---|---|---|---|
| ber. : | 40.3 | 2.0 | 6.3 | 7.2 | 7.9 | 25.5 |
| gef. : | 40.4 | 2.1 | 6.3 | 7.8 | 8.0 | 24.1 |

Analog können beispielsweise folgende Verbindungen synthetisiert werden :

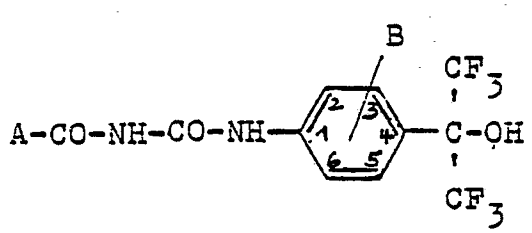

| Nr. | A | B(=$R_n$) | Fp (°C) |
|---|---|---|---|
| 3 | 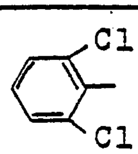 | - | 221-224 |
| 4 | 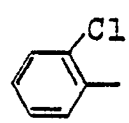 | - | 175-177 |
| 5 | 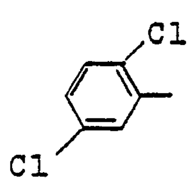 | - | 200-202 |

| Nr. | A | B(=$R_{II}$) | Fp (°C) |
|---|---|---|---|
| 6 | 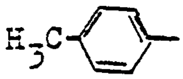 | - | 225-231 |
| 7 | 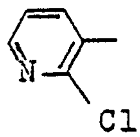 | - | 196-198 |
| 8 | 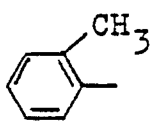 | - | 208-213 |
| 9 | 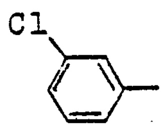 | - | 199-201 |
| 10 | 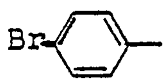 | - | 197-201 |
| 11 | 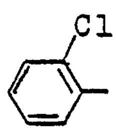 | 2-Methyl | 175-177 |
| 12 | 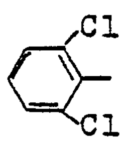 | 2,5-Dimethyl | 230-232 |
| 13 | 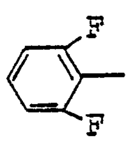 | 2,6-Dimethyl | 229-232 |
| 14 | 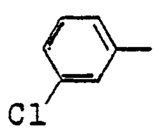 | 2,6-Dimethyl | 214-217 |
| 15 | 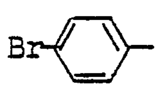 | 2,6-Dimethyl | 242-244 |
| 16 | 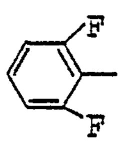 | 3-Methyl | 139-142 |

| Nr. | A | B(=$R_n$) | Fp (°C) |
|---|---|---|---|
| 17 | 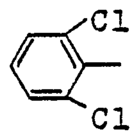 | 3-Methyl | 121-123 |
| 18 | 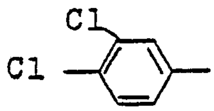 | | 199-203 |
| 19 | 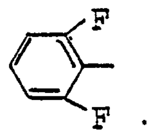 | 2-Methoxy | 195-197 |
| 20 | 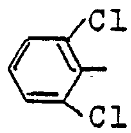 | 2,6-Dimethyl | 233-237 |
| 21 | 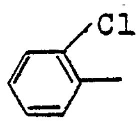 | 2,6-Dimethyl | 199-203 |
| 22 | 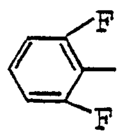 | 2,5-Dimethyl | 228-231 |
| 23 | 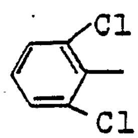 | 2-Methyl | 212-218 |
| 24 | 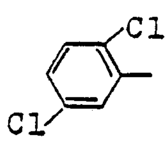 | 2-Methyl | 223-226 |
| 25 | 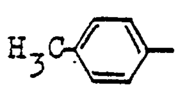 | 2-Methyl | 211-213 |
| 26 | 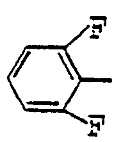 | 2-Methyl | 171-174 |
| 27 | 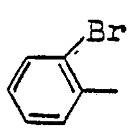 | - | 194 |

| Nr. | A | B(=$R_n$) | Fp (°C) |
|---|---|---|---|
| 28 | 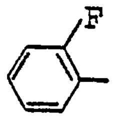 | - | 150-154 |
| 29 | 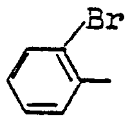 | 2-Methyl | 168-171 |
| 30 | 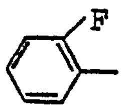 | 2-Methyl | 172-176 |
| 31 | 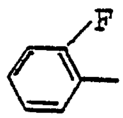 | 2-Ethyl | 135-137 |
| 32 | 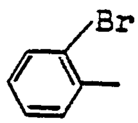 | 2-Ethyl | 162-168 |
| 33 | 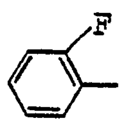 | 2-Isopropyl | 149-156 |
| 34 | 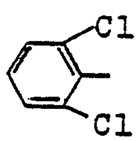 | 2-Ethyl | 205-208 |
| 35 | 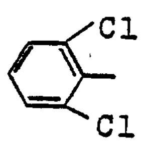 | 2-Isopropyl | 156-160 |
| 36 | 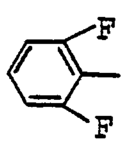 | 2-Ethyl | 175-179 |
| 37 | 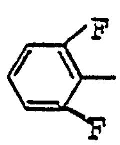 | 2-Isopropyl | 140-143 |

| Nr. | A | B(=$R_n$) | Fp ($^{o}C$) |
|---|---|---|---|
| 38 | 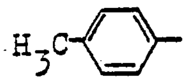 | 2,5-Dimethyl | 187-189 |
| 39 | 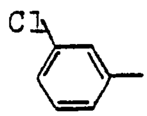 | 2-Methyl | 224-226 |
| 40 | 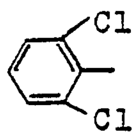 | 2-Methoxy | 135-138 |
| 41 | 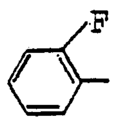 | 2-Methoxy | 164-167 |
| 42 | 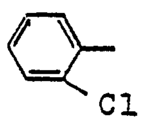 | 2-Methoxy | 163-166 |
| 43 | 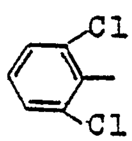 | 4-Methyl | 143-148 |
| 44 | 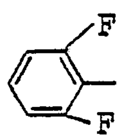 | 4-Methyl | 143-150 |
| 45 | 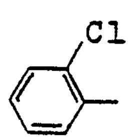 | 4-Methyl | 160-169 |
| 46 | 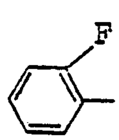 | 4-Methyl | 194-196 |

| Nr. | A | B(=$R_n$) | Fp (°C) |
|---|---|---|---|
| 47 | 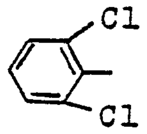 | 4-Ethyl | 170-178 |
| 48 | 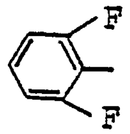 | 4-Ethyl | 98-106 |
| 49 | 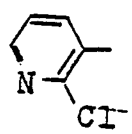 | 4-Methyl | 196-197 |
| 50 | 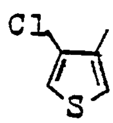 | 4-Methyl | 152-156 |
| 51 | 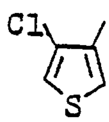 | 4-Ethyl | 150-158 |
| 52 | 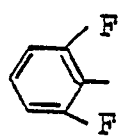 | 5-Methyl | 140-142 |
| 53 | 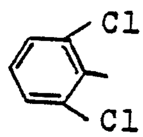 | 5-Methyl | 121-123 |

Die erfindungsgemäßen Verbindungen sind geeignet, Schädlinge aus der Klasse der Insekten und Milben wirksam zu bekämpfen.

Zu den schädlichen Insekten gehören aus der Ordnung der Schmetterlinge (Lepidoptera) beispielsweise Plutella maculipennis (Kohlschabe), Leucoptera coffeella (Kaffeemotte), Hyponomeuta malinellus (Apfelbaumgespinstmotte), Agyresthia conjugella (Apfelmotte), Sitotroga cerealella (Getreidemotte), Phthorimaea operculella (Kartoffelmotte), Capua reticlulana (Apfelschalenwickler), Sparganothis pilleriana (Springwurm), Cacoecia murinana (Tannentriebwickler), Tortrix viridana (Eichenwickler), Clysia ambiguella (Heu- und Sauerwurm), Evetria buoliana (Kieferntriebwickler), Polychrosis botrana (Bekreuzter Traubenwickler), Cydia pomonella (Obstmade), Laspeyresia molesta (Pfirsichtriebbohrer), Laspeyresia funebrana (Pflaumenwickler), Ostrinia nubilalis (Maiszünsler), Loxostege sticticalis (Rübenzünsler), Ephestia kuehniella (Mehlmotte), Chilo suppressalis (Reisstengelbohrer), Galleria mellonella (Wachsmotte), Malacosoma neustria (Ringelspinner), Dendrolimus pini (Kiefernspinner), Thaumatopoea pityocampa (Pinienprozessionsspinner), Phalera bucephala (Mondfleck), Cheimatobia brumata (Kleiner Frostspanner), Hibernia defoliaria (großer Frostspanner), Bupalus piniarius (Kiefernspanner), Hyphantria cunea (Weißer Bärenspinner), Agrotis segetum (Wintersaateule), Agrotis ypsilon (Ypsiloneule), Barathra brassicae (Kohleule), Cirphis unipuncta (Heerwurm), Prodenia litura (Baumwollraupe); Laphygma exigua (Rüben-Heerwurm), Panolis flammea (Fortleule), Earias insulana (Baumwollkapselwurm), Plusia gamma (Gammaeule), Alabama argillacea (Baumwollblattwurm), Lymantria dispar (Schwammspinner), Lymantria monachae (Nonne), Pieris brassicae (Kohlweißlung), Aporia crataegi (Baumweißling) ;

aus der Ordnung der Käfer (Coleoptera) beispielsweise Blitophaga undata (Schwarzer Rübenaaskäfer), Melanotus cummunis (Drahtwurm), Limonius californicus (Drahtwurm), Agriotes lineatus (Saatschnellkäfer), Agriotes obscurus (Humusschnellkäfer), Agrilus sinuatus (Birnbaum-Prachtkäfer), Meligethes aeneus (Rapsglanzkäfer), Atomaria linearis (Moosknopfkäfer), Epilachna varivestris (Mexikanischer Bohnenkäfer), Phyllopertha horticola (Junikäfer), Popillia japonica (Japankäfer), Melolontha melolontha (Feldmaikäfer), Melolontha hippocastani (Waldmaikäfer), Amphimallus solstitialis (Brachkäfer), Crioceris asparagi (Spargelhähnchen), Lema melanopus (Getreidehähnchen), Leptinotarsa decemlineata (Kartoffelkäfer), Phaedon cochleariae (Meerrettich-Blattkäfer), Phyllotreta nemorum (Kohlerdfloh), Chaetochnema tibialis (Rübenflohkäfer), Phylloides chrysocephala (Raps-Flohkäfer), Diabrotica 12-punctata (Südlicher Maiswurzelwurm), Cassida nebulosa (Nebliger Schildkäfer), Bruchus lentis (Linsenkäfer), Bruchus rufimanus (Pferdebohnenkäfer), Bruchus pisorum (Erbsenkäfer), Sitona lineatus (Linierter Blattrandkäfer), Otiorrhynchus sulcatus (Gefurchter Lappenrüßler), Otiorrhynchus ovatus (Erdbeerwurzelrüßler), Hylobies abietis (großer Brauner Rüsselkäfer), Byctiscus betulae (Rebenstecher), Anthonomus pomorum (Apfelblütenstecher), Anthonomus grandis (Kapselkäfer), Ceuthorrhynchus assimilis (Kohlenschotenrüßler), Ceuthorrhynchus napi (Großer Kohltriebrüßler), Sitophilus granaria (Kornkäfer), Anisandrus dispar (Ungleicher Holzborkenkäfer), Ips typographus (Buchdrucker), Blastophagus piniperda (Gefurchter Wandgärtner) und Schaben, wie die deutsche Schabe (Blatta germanica, die amerikanische Schabe (Periplaneta americana), die orientalische Schabe (Blatta orientalis), die Riesenschabe (Blaberus giganteus), die schwarze Riesenschabe (Blaberus fuscus) ;

aus der Ordnung der Zweiflügler (Diptera) beispielsweise Mayetiola destructor (Hessenfliege), Dasyneura brassicae (Kohlenschoten-Gallmücke), Contarinia tritici (Gelbe Weizen-Gallmücke), Haplodiplosis equestris (Sattelmücke), Tipula paludosa (Wiesenschnake), Tipula oleracea (Kohlschnake), Dacus cucurbitae (Melonenfliege), Dacus oleae (Olivenfliege), Ceratitis capitata (Mittelmeer-Fruchtfliege), Rhagoletis cerasi (Kirschfruchtfliege), Rhagoletis pomonella (Apfelmade), Anastrepha ludens (Mexikanische Fruchtfliege), Oscinella frit (Fritfliege), Phorbia coarctata (Brachfliege), Phorbia antiqua (Zwiebelfliege), Phorbia brassicae (kleine Kohlfliege), Pegomya hyoscyami (Rübenfliege) ;

aus der Ordnung der Hautflügler (Hymenoptera) beispielsweise Athalia rosae (Rübsenblattwespe), Hoplocampa minuta (Pflaumensäugewespe), Monomorium pharaonis (Pharaoameise), Solenopsis geminata (Feuerameise), Atta sexdens (Blattschneiderameise) ;

aus der Ordnung der Wanzen (Heteroptera) beispielsweise Nezara viridula (Grüne Reiswanze), Eurygaster integriceps (Asiatische Getreidewanze), Blissus leucopterus (Chinch bug.), Dysdercus cingulatus (Kapok-Wanze), Dysdercus intermedius (Baumwollwanze), Piesma quadrata (Rübenwanze), Lygus pratensis (Gemeine Wiesenwanze) ;

aus der Ordnung der Pflanzensauger (Homoptera) beispielsweise Perkinsiella saccharicida (Zuckerrohrzikade), Nilaparvata lugens (Braune Zikade), Empoasca fabae (Kartoffelzikade), Psylla mali (Apfelblattsauger), Psylla piri (Birnblattsauger), Trialeurodes vaporariorum (Weiße Fliege), Aphis fabae (Schwarze Bohnenlaus), Aphis pomi (Grüne Apfellaus), Aphis sambuci (Holunderblattlaus), Aphidula nasturtii (Kreuzdornblattlaus), Cerosipha gossypii (Gurkenblattlaus), Sappaphis mali (Rosige Apfellaus), Sappaphis mala (Mehlige Birnblattlaus), Dysphis radicola (Mehlige Apfelfalterlaus), Brachycaudus cardui (große Pflaumenblattlaus), Brevicoryne brassicae (Kohlblattlaus), Phorodon humuli (Hopfenblattlaus), Rhopalomyzus ascalonicus (Zwiebellaus), Myzodes persicae (Grüne Pfirsichlaus), Myzus cerasi (Schwarze Sauerkirschenlaus), Dysaulacorthum pseudosolani (Gefleckte Kartoffellaus), Acyrthosiphon onobrychis (Grüne Erbsenlaus), Macrosiphon rasae (Große Rosenblattlaus), Megoura viciae (Wickellaus), Schizoneura lanuginosa (Birnenblattlaus), Pemphigus bursarius (Salatwurzellaus), Dreyfusia nordmannianae (Tannentrieblaus), Dreyfusia piceae (Weißtannenstammlaus), Adelges laricis (Rote Fichtengallenlaus), Viteus vitifolii (Reblaus) und aus der Ordnung der Termiten (Isoptera) beispielsweise Reticulitermes lucifugus.

Zur Klasse der Milben (Acarina) gehören beispielsweise Ixodes ricinus (Holzbock), Ornithodorus moubata, Amblyomma americanum, Dermacentor silvarum, Boophilus microplus, Tetranychus telarius, Tetranychus atlanticus ; Tetranychus pacificus, Paratetranychus pilosus, Bryobia praetiosa.

Die erfindungsgemäßen Verbindungen könen mit Erfolg im Pflanzenschutz sowie auf dem Hygiene-, Vorratsschutz- und Veterinärsektor als Schädlingsbekämpfungsmittel eingesetzt werden.

Die Wirkstoffe können als solche, in Form ihrer Formulierungen oder den daraus bereiteten Anwendungsmöglichkeiten z.B. in Form von direkt versprühbaren Lösungen, Pulvern, Suspensionen oder Dispersionen, Emulsionen, Öldispersionen, Pasten, Stäubemitteln, Streumitteln, Granulaten durch Versprühen, Vernebeln, Verstäuben, Verstreuen oder Gießen angewendet werden. Die Anwendungsformen richten sich ganz nach den Verwendungszwecken ; sie sollten in jedem Fall möglichst die feinste Verteilung der erfindungsgemäßen Wirkstoffe gewährleisten.

Zur Herstellung von direkt versprühbaren Lösungen, Emulsionen, Pasten oder Öldispersionen kommen Mineralölfraktionen von mittlerem bis hohem Siedepunkt, wie Kerosin oder Dieselöl, ferner Kohlenteeröle, sowie Öle pflanzlichen oder tierischen Ursprungs, aliphatische, cyclische und aromatische Kohlenwasserstoffe, wie Benzol, Toluol, Xylol, Paraffin, Tetrahydronaphthalin, alkylierte Naphthaline oder deren Derivate, wie Methanol, Ethanol, Propanol, Butanol, Chloroform, Tetrachlorkohlenstoff, Cyclohexanol, Cyclohexanon, Chlorbenzol, Isophoron, stark polare Lösungsmittel, wie Dimethylformamid, Dimethylsulfoxid, N-Methylpyrrolidon, Wasser, in Betracht.

Wäßrige Anwendungsformen können aus Emulsionskonzentraten, Pasten oder netzbaren Pulvern (Spritzpulvern, Öldispersionen) durch Zusatz von Wasser bereitet werden. Zur Herstellung von Emulsionen, Pasten oder Öldispersionen können die Substanzen als solche oder in einem Öl oder Lösungsmittel gelöst, mittels Netz-, Haft-, Dispergier- oder Emulgiermittel in Wasser homogenisiert werden. Es können aber auch aus wirksamer Substanz Netz-, Haft-, Dispergier- oder Emulgiermittel und eventuell Lösungsmittel oder Öl bestehende Konzentrate hergestellt werden, die zur Verdünnung mit Wasser geeignet sind.

Als oberflächenaktive Stoffe kommen Alkali-, Erdalkali-, Ammoniumsalze von Ligninsulfonsäure, Naphthalinsulfonsäure, Phenolsulfonsäure, Alkylarylsulfonate, Alkylsulfate, Alkylsulfonate, Alkali- und Erdalkalisalze der Dibutylnaphthalinsulfonsäure, Laurylethersulfat, Fettalkoholsulfate, fettsaure Alkali- und Erdalkalisalze, Salze sulfatierter Hexadecanole, Heptadecanole, Octadecanole, Salze von sulfatiertem Fettalkoholglykolether, Kondensationsprodukte von sulfoniertem Naphthalin und Naphthalinderivaten mit Formaldehyd, Kondensationsprodukte des Naphthalins bzw. der Naphthalinsulfonsäuren mit Phenol und Formaldehyd, Polyoxyethylenoctylphenolether, ethoxyliertes Isooctylphenol, Octylphenol, Nonylphenon, Alkylphenolpolyglykolether, Tributylphenylpolyglykolether, Alkylarylpolyetheralkohole, Isotridecylalkohol, Fettalkoholethylenoxid-Kondensate, ethoxyliertes Rizinusöl, Polyoxyethylenakylether, ethoxyliertes Polyoxypropylen, Laurylalkoholpolyglykoletheracetal, Sorbitester, Lignin, Sulfitablaugen und Methylcellulose in Betracht.

Pulver-, Streu- und Stäubemittel können durch Mischen oder gemeinsames Vermahlen der wirksamen Substanzen mit einem festen Trägerstoff hergestellt werden.

Granulate, z.B. Umhüllungs-, Imprägnierungs- und Homogengranulate, können durch Bindung der Wirkstoffe an feste Trägerstoffe hergestellt werden. Feste Trägerstoffe sind z.B. Mineralerden, wie Silicagel, Kieselsäuren ; Kieselgele, Silikate, Talkum, Kaolin, Attaclay, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie z.B. Ammoniumsulfat, Ammoniumphosphat, Ammoniumnitrat, Harnstoffe und pflanzliche Produkte, wie Getreidemehl, Baumrinden-, Holz- und Nußschalenmehl, Cellulosepulver und andere feste Trägerstoffe.

Die Formulierungen enthalten im allgemeinen zwischen 0,1 und 95 Gewichtsprozent Wirkstoff, vorzugsweise zwischen 0,5 und 90 Gewichtsprozent.

Die Wirkstoffkonzentrationen in den anwendungsfertigen Zubereitungen können in größeren Bereichen variiert werden. Im allgemeinen liegen sie zwischen 0,000 1 und 10 %, vorzugsweise zwischen 0,01 und 1 %.

Die Wirkstoffe können auch mit gutem Erfolg im Ultra-Low-Volume-Verfahren (ULV) verwendet werden, wobei es möglich ist, Formulierungen mit über 95 Gewichtsprozent Wirkstoff oder sogar den 100 %igen Wirkstoff allein auszubringen.

Beispiele für Formulierungen sind :

I. 3 Gewichtsteile N-(2-Chlorbenzoyl)-N'-[4-(1-hydroxy-1-trifluormethyl-2,2,2-trifluor-ethyl)-phenyl]-harnstoff werden mit 97 Gewichtsteilen feinteiligem Kaolin innig vermischt. Man erhält auf diese Weise ein Stäubemittel, das 3 Gewichtsprozent des Wirkstoffs enthält.

II. 30 Gewichtsteile N-(2,6-Dichlorbenzoyl)-N'-[4-(1-hydroxy-1-trifluormethyl-2,2,2-trifluor-ethyl)-phenyl]-harnstoff werden mit einer Mischung aus 92 Gewichtsteilen pulverförmigem Kieselsäuregel und 8 Gewichtsteilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.

III. 20 Gewichtsteile N-(2,4-Difluorbenzoyl)-N'-[4-(1-hydroxy-1-trifluormethyl-2,2,2-trifluor-ethyl)-phenyl]-harnstoff werden in einer Mischung gelöst, die aus 80 Gewichtsteilen Xylol, 10 Gewichtsteilen des Anlagerungsproduktes von 8 bis 10 Mol Ethylenoxid an 1 Mol Ölsäure-N-monoethanolamid, 5 Gewichtsteilen Calciumsalz der Dodecylbenzolsulfonsäure und 5 Gewichtsteilen des Anlagerungsproduktes von 40 Mol Ethylenoxid an 1 Mol Ricinusöl besteht. Durch Ausgießen und feines Verteilen der Lösung in 100 000 Gewichtsteilen Wasser erhält man eine wäßrige Dispersion, die 0,02 Gewichtsprozent des Wirkstoffs enthält.

IV. 20 Gewichtsteile N-(4-Brombenzoyl)-N'-[4-(1-hydroxy-1-trifluormethyl-2,2,2-trifluor-ethyl)-2,6-dimethylphenyl]-harnstoff werden in einer Mischung gelöst, die aus 40 Gewichtsteilen Cyclohexanon, 30 Gewichtsteilen Isobutanol, 20 Gewichtsteilen des Anlagerungsproduktes von 7 Mol Ethylenoxid an 1 Mol Isooctylphenol und 10 Gewichtsteilen des Anlagerungsproduktes von 40 Mol Ethylenoxid an 1 Mol Ricinusöl besteht. Durch Eingießen und feines Verteilen der Lösung in 100 000 Gewichtsteilen Wasser erhält man eine wäßrige Dispersion, die 0,02 Gewichtsprozent des Wirkstoffs enthält.

Zu den Einzelwirkstoffen können Öle verschiedenen Typs, Herbizide, Fungizide, andere Insektizide,

Bakterizide, gegebenenfalls auch erst unmittelbar vor der Anwendung (Tankmix), zugesetzt werden. Diese Mittel können zu den erfindungsgemäßen Mitteln im Gewichtsverhältnis 1:10 bis 10:1 zugemischt werden.

Beispielsweise können folgende Mittel zugemischt werden : 1,2-Dibrom-3-chlorpropan, 1,3-Dichlorpropen, 1,3-Dichlorpropen + 1,2-Dichlorpropan, 1,2-Dibrom-ethan, 2-sec.-Butyl-phenyl-N-methylcarbamat, 0-Chlorphenyl-N-methylcarbamat, 3-Isopropyl-5-methylphenyl-N-methylcarbamat, 0-Isopropoxyphenyl-N-methylcarbamat, 3,5-Dimethyl-4-methylmercapto-phenyl-N-methylcarbamat, 4-Dimethylamino-3,5-xylyl-N-methylcarbamat, 2-(1,3-Dioxolan-2-yl)-phenyl-N-methylcarbamat, 1-Naphthyl-N-methylcarbamat, 2,3-Dihydro-2,2-dimethyl-benzofuran-7-yl-N-methylcarbamat, 2,2-Dimethyl-1,3-benzodioxol-4-yl-N-methylcarbamat, 2-Dimethylamino-5,6-dimethyl-4-pyrimidinyl-dimethylcarbamat, 2-Methyl-2-(methylthio)-propionaldehyd-0-(methylcarbamoyl)-oxim, S-Methyl-N-(methylcarbamoyl)-oxy-thio-acetimidat, Methyl-N',N'-dimethyl-N-(methylcarbamoyl)oxy-1-thiooxamidat, N-(2-Methyl-4-chlor-phenyl)-N',N'-dimethylformamidin, Tetrachlorthiophen, 0,0-Dimethyl-0-(p-nitrophenyl)-phosphorthioat, 0,0-Diethyl-0-(p-nitrophenyl)-phosphorthioat, 0-Äthyl-0-(p-nitrophenyl)-phenyl-phosphonothioat, 0,0-Dimethyl-0-(3-methyl-4-nitrophenyl)-phosphorthioat, 0,0-Diethyl-0-(2,4-dichlorphenyl)-phosphorthioat, 0-Ethyl-0-(2,4-dichlorphenyl)-phenyl-phosphonothioat, 0,0-Dimethyl-0-(2,4,5-trichlorphenyl)-phosphorthioat, 0-Ethyl-0-(2,4,5-trichlorphenyl)-ethylphosphonothioat, 0,0-Dimethyl-0-(4-brom-2,5-dichlorphenyl)-phosphorthioat, 0,0-Dimethyl-0-(2,5-dichlor-4-jodphenyl)-phosphorthioat, 0,0-Dimethyl-0-(3-methyl-4-methylthiophenyl)-phosphorthioat, 0-Ethyl-0-(3-methyl-4-methylthiophenyl)-isopropyl-phosphoramidat, 0,0-Diethyl-0-[p-(methylsulfinyl)phenyl]-phosphorthioat, 0-Ethyl-S-phenyl-ethyl-phosphordithioat, 0,0-Diethyl-[2-chlor-1-(2,4-dichlorphenyl)-vinyl]-phosphat, 0,0-Dimethyl-[2-chlor-1-(2,4,5-trichlorphenyl)]-vinyl-phosphat, 0,0-Dimethyl-S-(1-phenyl)-ethylacetat-phosphordithioat, Bis-(dimethylamino)-fluorphosphinoxid, Octamethyl-pyrophosphoramid, 0,0,0,0-Tetraethyldithio-pyrophosphat, S-Chlormethyl-0,0-diethyl-phosphordithioat, 0-Ethyl-S,S-dipropyl-phosphordithioat, 0,0-Dimethyl-0,2,2-dichlorvinyl-phosphat, 0,0-Dimethyl-1,2-dibrom-2,2-dichlorethylphosphat, 0,0-Dimethyl-2,2,2-trichlor-1-hydroxy-ethylphosphonat, 0,0-Dimethyl-S-[1,2-biscarbethoxy-äthyl-(1)]-phosphordithioat, 0,0-Dimethyl-0-(1-methyl-2-carbmethoxy-vinyl)-phosphat, 0,0-Dimethyl-S-(N-methyl-carbamoyl-methyl)-phosphordithioat, 0,0-Dimethyl-S-(N-methyl-carbamoyl-methyl)-phosphorthioat, 0,0-Dimethyl-S-(N-methoxyethyl-carbamoyl-methyl)-phosphordithioat, 0,0-Dimethyl-S-(N-formyl-N-methyl-carbamoylmethyl)-phosphordithioat, 0,0-Dimethyl-0-[1-methyl-2-(methyl-carbamoyl)-vinyl]-phosphat, 0,0-Dimethyl-0-[(1-methyl-2-dimethylcarbamoyl)-vinyl]-phosphat, 0,0-Dimethyl-0-[(1-methyl-2-chlor-2-diethylcarbamoyl)-vinyl]-phosphat, 0,0-Diethyl-S-(ethylthio-methyl)-phosphordithioat, 0,0-Diethyl-S-[(p-Chlorphenylthio)-methyl]-phosphordithioat, 0,0-Dimethyl-S-(2-ethylthioethyl)-phosphorthioat, 0,0-Dimethyl-S-(2-ethylthioethyl)-phosphordithioat, 0,0-Dimethyl-S-(2-ethylsulphinyl-ethyl)-phosphorthioat, 0,0-Diethyl-S-(2-ethylthioethyl)-phosphordithioat, 0,0-Dimethyl-S-(2-ethylsulphinyl-ethyl)-phosphorthioat, 0,0-Diethylthiophosphoryl-iminophenyl-acetonitril, 0,0-Diethyl-S-(2-chlor-1-phthalimidoethyl)-phosphordithioat, 0,0-Diethyl-S-[6-chlor-benzoxazolon-(2)-yl(3)]-methyldithiophosphat, 0,0-Dimethyl-S-[2-methoxy-1,3,4-thiodiazol-5-onyl(4)-methyl]-phosphordithioat, 0,0-Diäthyl-0-[3,5,6-trichlor-pyridyl-(2)]-phosphorthioat, 0,0-Diethyl-0-(2-pyrazinyl)-phosphorthioat, 0,0-Diethyl-0-[2-isopropyl-4-methyl-pyrimidinyl(6)]-phosphorthioat, 0,0-Diethyl-0-[2-(diethylamino)-6-methyl-4-pyrimidinyl]-thionophosphat, 0,0-Dimethyl-S-(4-oxo-1,2,3-benzotriazin-3-yl-methyl)-phosphordithioat, 0,0-Dimethyl-S-[(4,6-diamino-1,3,5-triazin-2-yl)-methyl]-phosphordithioat, 0,0-Diethyl-(1-phenyl-1,2,4-triazol-3-yl)-thionophosphat, 0,S-Dimethyl-phosphor-amido-thioat, 0,S-Dimethyl-N-acetyl-phosphoramidothioat, γ-Hexachlorcyclohexan, 1,1-Di-(p-methoxyphenyl)-2,2,2-trichlor-ethan, 6,7,8,9,10,10-Hexachloro-1,5,5a,6,9,9a-hexahydro-6,9-methano-2,4,3-benzodioxa-thiepin-3-oxid, Pyrethrine, DL-2-Allyl-3-methyl-cyclopenten-(2)-on-(1)-yl-(4)-DL-cis, trans-chrysanthemat, 5-Benzyl-furyl-(3)-methyl-DL-cis, trans-chrysanthemat, 3-Phenoxybenzyl(±)-cis, trans-2,2-dimethyl-3-(2,2-dichlorvinyl)-cyclopropancarboxylat, α-Cyano-3-phenoxybenzyl(±)-cis, trans-2,2-dimethyl-3-(2,2-dichlorvinyl)-cyclopropancarboxylat, (s)-α-Cyano-3-phenoxybenzylcis(1R, 3R)-2,2-dimethyl-3-(2,2-dibromvinyl)-cyclopropancarboxylat, 3,4,5,6-Tetrahydrophthalimidoäthyl-DL-cis,-trans-chrysanthemat, 2-Methyl-5-(2-propinyl)-3-furylmethyl-chrysanthemat, α-Cyano-3-phenoxybenzyl-α-isopropyl-4-chlorphenylacetat.

Die folgenden Beispiele belegen die biologische Wirkung der neuen Verbindungen. Vergleichsmittel sind N-(2,6-Difluorbenzoyl)-N'-(4-chlorphenyl)-harnstoff (I ; DE-OS 2 123 236) und N-(2,6-Difluorbenzoyl)-N'-(5-chlor-pyridinyl-2)-harnstoff (II ; J. Agric. Food Chem. *26*, 164-166 (1978)). Die Numerierung der Wirkstoffe entspricht den Herstellungsbeispielen und der tabellarischen Aufzählung.

## Beispiel A

Versuche mit gezählten Weibchen der Spinnmilbe (Tetranychus telarius).

Getopfte Buschbohnen werden in der Spritzkabine auf dem Drehteller mit der wässrigen Wirkstoffaufbereitung tropfnaß gespritzt. Nach dem Abtrocknen stanzt man mit einem Korkbohrer Blattstücke von 25 mm Durchmesser aus den Spreiten. Sie werden auf Zellstoffstücke gelegt, welche an den Seiten im Wasser hängen.

Die Blattstanzstücke werden mit je 10 adulten Weibchen belegt. Nach 6 Tagen erfolgt die Auswertung, wobei Eier, Larven und Adulte ausgezählt werden.

| Wirkstoff Nr. | Konzentration der Wirkstoffaufbereitung (Gew.-%) | innerhalb von 6 Tagen abgelegte Eier | davon geschlüpft |
|---|---|---|---|
| 1 | 0,1 | 5 | 0 |
| | 0,05 | 7 | 0 |
| | 0,02 | 35 | 0 |
| | 0,01 | 35 | 0 |
| 2 | 0,1 | 3 | 0 |
| | 0,05 | 4 | 0 |
| | 0,025 | 7 | 0 |
| 3 | 0,1 | 5 | 2 |
| 4 | 0,05 | 2 | 0 |
| 5 | 0,1 | 17 | 0 |
| | unbehandelt | 75 | 55 |

Beispiel B

Wirkung auf Larven der Mehlmotte (Ephestia kuehniella) Zuchtversuch.

Weizenmehl, welches stark mit Eiern der Mehlmotte belegt ist, wird mit den Wirkstoffen innig gemischt. Davon werden 10 g in Gläser von 250 ml Volumen abgefüllt und bei 22 °C gelagert.

Nach 4 Wochen beurteilt man die Entwicklung der Larven.

| Wirkstoff Nr. | Konzentration des Wirkstoffs im Mehl (ppm) | |
|---|---|---|
| 1 | 4 | starke Entwicklungshemmung |
| I | 100 | Entwicklungshemmung |
| I | 50 | unwirksam |

Beispiel C

Zuchtversuch mit Moskito-Larven (Aedes aegypti).

200 ml Leitungswasser werden mit der Wirkstoffaufbereitung versetzt und darauf mit 30 bis 40 Aedes-Larven im 4. Stadium belegt.

Die Versuchstemperatur beträgt 25 °C. Beurteilt werden Verpuppung und Schlüpfen der Imagines, wobei eine unbehandelte Kontrolle als Maßstab dient.

Während der Versuchsdauer wird einmal gefüttert.

| Wirkstoff Nr. | Konzentration der Wirkstoffaufbereitung (ppm) | Mortalitätsrate (%) |
|---|---|---|
| 1 | 0,01 | 100 |
| 3 | 0,1 | 100 |
| | 0,05 | starke Hemmung |
| 4 | 0,05 | 100 |
| 5 | 0,5 | 100 |

Beispiel D

Zuchtversuch mit Tribolium castaneum.

Je 10 g Weizenmehl werden mit der Wirkstoff-Formulierung sorgfältig gemischt und in 250 ml-Glasflaschen abgefüllt. Man belegt jede Flasche mit 20 Käfern zur Eiablage. Nach 14 Tagen werden die Käfer abgesiebt. Das behandelte Mehl mit den Eiablagen bleibt bei 24 °C zur Beobachtung stehen, bis die nächste Käfergeneration schlüpft.

| Wirkstoff Nr. | Konzentration des Wirkstoffs im Mehl (ppm) | |
|---|---|---|
| 1 | 2,0 | totale Hemmung |
| | 1,0 | starke Hemmung |
| | 0,5 | Hemmung |
| I | 2,0 | Hemmung |
| | 1,0 | unwirksam |
| II | 100 | unwirksam |

Beispiel E

Zuchtversuch mit Drosophila melanogaster.

40 ml eines Kleie-Agar-Nährbodens werden bei 60 °C in Plastikflaschen von 250 ml Volumen abgefüllt und darauf mit 2 ml der wäßrigen Wirkstoffaufbereitung innig vermischt.

Nach dem Erkalten beimpft man den Nährboden mit einer Hefesuspension und gibt eine Filterpapierrolle zu. Anschließend setzt man 20 bis 40 ca. 6 Tage alte Drosophila ein und verschließt die Gefäße.

Die Auswertung erfolgt nach 10 Tagen.

| Wirkstoff Nr. | Konzentration des Wirkstoffs im Nährboden (ppm) | |
|---|---|---|
| 1 | 5,0 | Entwicklungshemmung |
| 3 | 12,5 | Entwicklungshemmung |
| 4 | 25,0 | Entwicklungshemmung |
| II | 50,0 | Entwicklungshemmung |
| | 25,0 | unwirksam |

**Ansprüche für die Vertragsstaaten :** BE, CH, DE, FR, GB, IT, LU, NL, SE

1. Aroylharnstoffe der Formel I

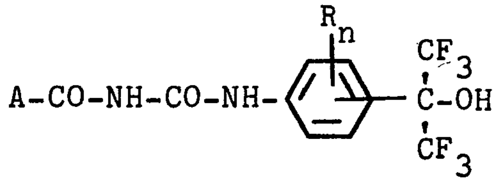

(I)

in der

A einen durch Halogen, unverzweigtes oder verzweigtes Alkyl mit 1 bis 4 Kohlenstoffatomen oder unverzweigtes oder verzweigtes Alkoxy mit 1 bis 4 Kohlenstoffatomen einfach oder mehrfach substituierten Phenyl-, Thienyl- oder Pyridinyl-3-rest,

R unverzweigtes oder verzweigtes Alkyl mit 1 bis 4 Kohlenstoffatomen oder unverzweigtes oder verzweigtes Alkoxy mit 1 bis 4 Kohlenstoffatomen und

n 0, 1 oder 2 bedeuten.

2. Aroylharnstoffe der Formel I gemäß Anspruch 1, *dadurch gekennzeichnet,* daß A für einen durch Halogen einfach oder zweifach substituierten Phenyl-, Thienyl- oder Pyridinyl-3-rest und n für 0 steht.

3. Verfahren zur Herstellung von Aroylharnstoffen der Formel I gemäß Anspruch 1, *dadurch gekennzeichnet,* daß man Isocyanate der Formel II

A—CO—NCO (II)

in der A die in Anspruch 1 genannten Bedeutungen hat, mit substituierten Anilinen der Formel III

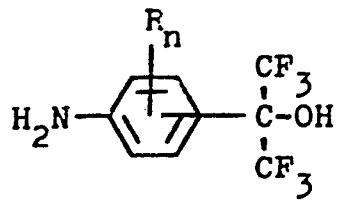

(III)

in der R und n die in Anspruch 1 genannten Bedeutungen haben, in Gegenwart eines inerten organischen Lösungsmittels bei einer Temperatur im Bereich zwischen 0 und 80 °C umsetzt.

4. Insektizide und akarizide Mittel, *gekennzeichnet durch* einen Gehalt an Aroylharnstoffen der Formel I gemäß Anspruch 1.

5. Insektizide und akarizide Mittel, enthaltend einen festen oder flüssigen inerten Trägerstoff und mindestens einen Aroylharnstoff der Formel I gemäß Anspruch 1.

6. Verfahren zur Bekämpfung von Insekten und Milben, *dadurch gekennzeichnet,* daß man Aroylharnstoffe der Formel I gemäß Anspruch 1 auf die genannten Schädlinge bzw. deren Lebensraum einwirken läßt.

7. Verfahren zur Herstellung von insektiziden und akariziden Mitteln gemäß Anspruch 5, *dadurch gekennzeichnet,* daß man Aroylharnstoffe der Formel I mit einem flüssigen oder festen inerten Trägerstoff mischt.

**Ansprüche für den Vertragsstaat :** AT

1. Verfahren zur Herstellung von Aroylharnstoffen der Formel I

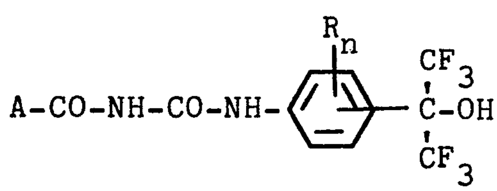

(I)

in der

A einen durch Halogen, unverzweigtes oder verzweigtes Alkyl mit 1 bis 4 Kohlenstoffatomen oder unverzweigtes oder verzweigtes Alkoxy mit 1 bis 4 Kohlenstoffatomen einfach oder mehrfach substituierten Phenyl-, Thienyl-oder Pyridinyl-3-rest,

R unverzweigtes oder verzweigtes Alkyl mit 1 bis 4 Kohlenstoffatomen oder unverzweigtes oder verzweigtes Alkoxy mit 1 bis 4 Kohlenstoffatomen und

n 0, 1 oder 2 bedeuten, *dadurch gekennzeichnet,* daß man Isocyanate der Forme II

A—CO—NCO (II)

in der A dasselbe wie oben bedeutet, mit substituierten Anilinen der Formel III

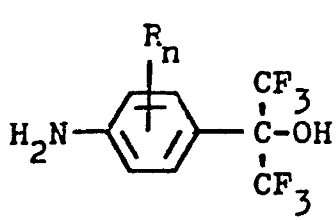

(III)

in der R und n dasselbe wie oben bedeuten, in Gegenwart eines inerten organischen Lösungsmittels bei einer Temperatur im Bereich zwischen 0 und 80 °C umsetzt.

2. Insektizide und akarizide Mittel, *gekennzeichnet durch* einen Gehalt an Aroylharnstoffen der Formel I gemäß Anspruch 1.

3. Insektizide und akarizide Mittel, enthaltend einen festen oder flüssigen inerten Trägerstoff und mindestens einen Aroylharnstoff der Formel I gemäß Anspruch 1.

4. Verfahren zur Bekämpfung von Insekten und Milben, *dadurch gekennzeichnet,* daß man Aroylharnstoffe der Formel I gemäß Anspruch 1 auf die genannten Schädlinge bzw. deren Lebensraum einwirken läßt.

5. Verfahren zur Herstellung von insektiziden und akariziden Mitteln gemäß Anspruch 3, *dadurch gekennzeichnet,* daß man Aroylharnstoffe der Formel I mit einem flüssigen oder festen inerten Trägerstoff mischt.

**Claims for the Contracting States :** BE, CH, DE, FR, GB, IT, LU, NL, SE

1. Aroylureas of the formula I

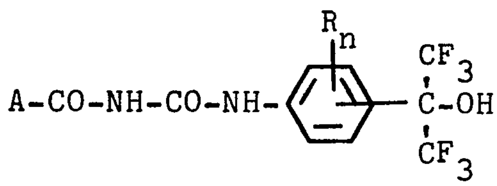

(I)

where

A denotes a phenyl, thienyl or pyridyl-3 radical which is mono- or polysubstituted by halogen, linear or branched alkyl of 1 to 4 carbon atoms, or linear or branched alkoxy of 1 to 4 carbon atoms,

R denotes linear or branched alkyl of 1 to 4 carbon atoms or linear or branched alkoxy of 1 to 4 carbon atoms and

n denotes 0, 1 or 2.

2. Aroylureas of the formula I as claimed in claim 1, *characterized in that* A denotes a phenyl, thienyl or pyridyl-3 radical which is mono- or disubstituted by halogen and n is 0.

3. A process for the production of aroylureas of the formula I as claimed in claim 1, *characterized in that* isocyanates of the formula II

$$A—CO—NCO \quad (II)$$

where A has the meanings given in claim 1 are reacted with substituted anilines of the formula III

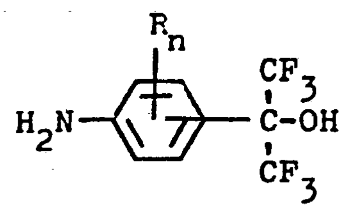

(III)

where R and n have the meanings given in claim 1, in the presence of an inert organic solvent at a temperature in the range from 0° to 80 °C.

4. Insecticides and acaricidal agents *characterized by* a content of aroylureas of the formula I as claimed in claim 1.

5. Insecticides and acaricidal agents containing a solid or liquid inert carrier and at least one aroylurea of the formula I as claimed in claim 1.

6. A process for combating insects and mites, *characterized in that* aroylureas of the formula I as claimed in claim 1 are allowed to act on the said pests or on their habitat.

7. A process for the production of insecticidal and acaricidal agents as claimed in claim 5, *characterized in that* aroylureas of the formula I are mixed with a liquid or solid inert carrier.

**Claims for the Contracting State :** AT

1. A process for the production of aroylureas of the formula I

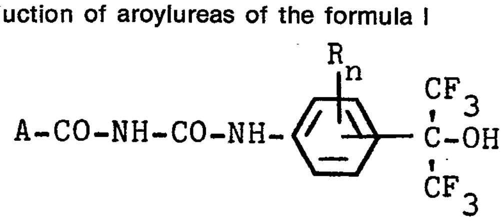

(I)

where

A denotes a phenyl, thienyl or pyridyl-3 radical which is mono- or polysubstituted by halogen, linear or branched alkyl of 1 to 4 carbon atoms, or linear or branched alkoxy of 1 to 4 carbon atoms,

R denotes linear or branched alkyl of 1 to 4 carbon atoms or linear or branched alkoxy of 1 to 4 carbon atoms and

n denotes 0, 1 or 2, *characterized in that* isocyanates of the formula II

A—CO—NCO (II)

where A has the same meanings as above are reacted with substituted anilines of the formula III

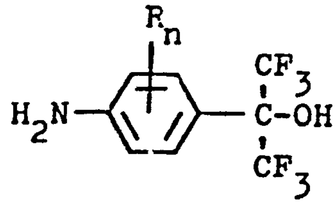

(III)

where R and n have the same meanings as above, in the presence of an inert organic solvent at a temperature in the range from 0° to 80 °C.

2. Insecticides and acaricidal agents *characterized by* a content of aroylureas of the formula I as claimed in claim 1.

3. Insecticides and acaricidal agents containing a solid or liquid inert carrier and at least one aroylurea of the formula I as claimed in claim 1.

4. A process for combating insects and mites, *characterized in that* aroylureas of the formula I as claimed in claim 1 are allowed to act on the said pests or on their habitat.

5. A process for the production of insecticidal and acaricidal agents as claimed in claim 3, *characterized in that* aroylureas of the formula I are mixed with a liquid or solid inert carrier.

**Revendications pour les Etats contractants :** BE, CH, DE, FR, GB, IT, LU, NL, SE

1. Aroylurées de formule I

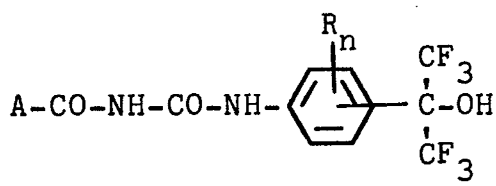

(I)

dans laquelle

A représente un reste phényle-, thiényle- ou pyridinyle-3, substitué une fois ou plusieurs fois par un halogène, un alkyle ramifié ou non en $C_1$ à $C_4$ ou un alcoxy ramifié ou non en $C_1$ à $C_4$,

R représente un alkyle ramifié ou non en $C_1$ à $C_4$ ou un alcoxy ramifié ou non en $C_1$ à $C_4$,

*n* est 0, 1 ou 2.

2. Aroylurées de formule I selon la revendication 1, dans lesquelle A représente un reste phényle-, thiényle-, ou pyridinyle-3 substitué une fois ou plusieurs fois par un halogène et *n* est zéro.

3. Procédé de préparation d'aroylurées de formule I selon la revendication 1, caractérisé par le fait qu'on fait réagir, en présence d'un solvant organique inerte, à une température comprise entre 0 et 80°C, des isocyanates de formule II

A—CO—NCO (II)

dans laquelle A a la signification indiquée plus haut, avec des anilines substituées de formule III

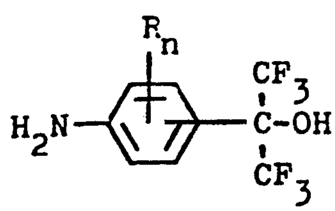

(III)

dans laquelle R et *n* ont les significations indiquées plus haut.

4. Insecticides et acaricides, caractérisés par le fait qu'ils contiennent des aroylurées de formule I selon la revendication 1.

5. Insecticides et acaricides contenant un support inerte solide ou liquide et au moins une aroylurée de formule I selon la revendication 1.

6. Procédé de lutte contre les insectes et les mites, caractérisé par le fait qu'on fait agir les aroylurées de formule I selon la revendication 1 sur les dits parasites ou leur biotope.

7. Procédé de préparation d'insecticides et acaricides selon la revendication 5, caractérisé par le fait qu'on mélange les aroylurées de formule I avec un support inerte liquide ou solide.

**Revendications pour l'Etat contractant :** AT

1. Procédé de préparation d'aroylurées de formule I

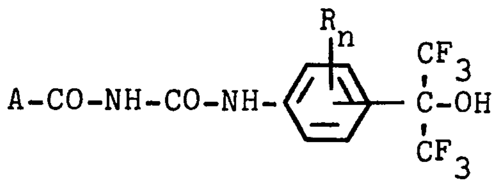

(I)

dans laquelle

A représente un reste phényle-, thiényle- ou pyridinyle-3, substitué une fois ou plusieurs fois par un halogène, un alkyle ramifié ou non, en $C_1$ à $C_4$ ou un alcoxy ramifié ou non, en $C_1$ à $C_4$,

R représente un alkyle ramifié ou non, en $C_1$ à $C_4$ ou un alcoxy ramifié ou non en $C_1$ à $C_4$,

*n* est 0, 1 ou 2, caractérisé par le fait qu'on fait réagir, en présence d'un solvant organique inerte, à une température comprise entre 0 et 80°C, des isocyanates de formule II

$$A{-}CO{-}NCO \qquad (II)$$

dans laquelle A a la signification indiquée plus haut, avec des anilines substituées de formule III

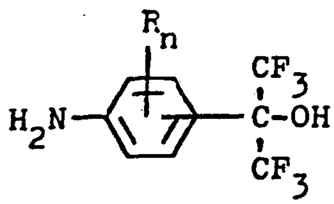

(III)

dans laquelle R et *n* ont les significations indiquées plus haut.

2. Insecticides et acaricides, caractérisés par le fait qu'ils contiennent des aroylurées de formule I selon la revendication 1.

3. Insecticides et acaricides contenant un support inerte solide ou liquide et au moins une aroylurée de formule I selon la revendication 1.

4. Procédé de lutte contre les insectes et les mites, caractérisé par le fait qu'on fait agir des aroylurées de formule I selon la revendication 1 sur les dits parasites ou leur biotope.

5. Procédé de préparation d'insecticides et acaricides selon la revendication 3, caractérisé par le fait qu'on mélange des aroylurées de formule I avec un support inerte liquide ou solide.